# EUROPEAN PATENT APPLICATION

(11) **EP 4 763 239 A1**
(43) Date of publication of application: **24.06.2026**
(21) Application number: 24222021.8
(22) Date of filing: 20.12.2024
(51) Int. Cl.: A61M 1/00

(54) **CARRYCASE FOR PORTABLE MEDICAL PRODUCT**

(71) Applicant: Mölnlycke Health Care AB, 431 21 Mölndal (SE)
(72) Inventor: JAKOBSSON, Conny, 443 38 LERUM (SE); VALHAM, David, 426 76 VÄSTRA FRÖLUNDA (SE)
(74) Representative: Kransell & Wennborg KB

(57) **Abstract**

The present disclosure relates to a carrycase (1) for a portable medical product (10), the carrycase extends in a longitudinal direction (Y) and in a transverse direction (X), the carrycase comprises a carrycase body (2), a shoulder strap (3) for carrying the carrycase and an elongated tube-shaped portion (4) extending from the carrycase body, wherein the carrycase is made of an elastic material (6).

## Description

### TECHNICAL FIELD

The present disclosure relates to the field of carrycases for portable medical products.

### BACKGROUND

Portable medical products, such as medical devices, are widely used in various therapeutic and diagnostic application. Such devices include but are not limited to negative pressure wound therapy (NPWT) devices, insulin pumps and infusion pumps. These devices play a critical role in managing chronical conditions, facilitate wound healing by removing exudate, reducing oedema, promoting tissue regeneration and providing other essential medical functions. While their clinical benefits are well-documented, the use of portable NPWT devices presents significant psychosocial challenges for patients.

Despite their clinical benefits, the use of portable medical devices often presents significant psychosocial challenges for patients. A key issue is the stigma associated with the visibility of medical equipment and its components. For example, transparent or translucent containers used for collecting bodily fluids or delivering medication can make their contents visible to others, causing embarrassment and discomfort for patients. This visibility may reinforce perceptions of illness and lead to social withdrawal or reluctance to participate in daily activities.

Additionally, the bulkiness and medical appearance of many portable medical products, such as portable NPWT devices, further contribute to the stigma. Carrying visibly medical equipment in public serves as a constant reminder of the patient's condition and can attract unwanted attention. Patients often report feelings of self-consciousness and a loss of normalcy, which can negatively affect mental health and overall quality of life.

These challenges highlight the need for innovations that address the psychosocial impact of using portable medical products. Solutions that reduce the visibility of bodily fluids and improve the portability and aesthetic integration of these devices into everyday life would enhance patient acceptance and adherence to treatment. By mitigating the stigma associated with these devices, patients could achieve better emotional well-being while benefiting from the therapeutic advantages of NPWT.

### SUMMARY

In view of the above-mentioned problems, it is an object of the present disclosure to make available a carrycase for a portable medical product with improved portability while preserving full functionality of the medical product. Additionally, the carrycase aims to improve integration of the portable medical product into everyday life with an aesthetically pleasing design and furthermore ensure that the carrycase is easy to manufacture and cost-efficient to produce.

According to a first aspect, a carrycase for a portable medical product is provided. The carrycase extends in a longitudinal direction and in a transverse direction, the carrycase comprises a carrycase body, a shoulder strap for carrying the carrycase and an elongated tube-shaped portion having a central through opening extending from the carrycase body, wherein the carrycase is made of an elastic material.

The portable medical product may for example be a medical device or a medical accessory. The carrycase is typically suitable for a medical device or medical accessory comprising a tubing. The carrycase is particularly suitable for a negative pressure wound therapy (NPWT) device comprising a negative pressure pump and a tubing connected to the negative pressure pump. Other suitable medical products and accessories may be insulin pumps, infusion pumps, portable chemotherapy pump and portable dialysis devices.

The present invention is based on the realization that using portable medical products in public and/or in a home-environment can cause embarrassment and discomfort for patients. Visible medical products, such as medical devices or accessories, often reinforce perceptions of illness, leading to social withdrawal or reluctance to participate in daily activities. For instance, transparent or translucent containers for collecting wound exudate often make bodily fluids visible to others. Additionally, the bulkiness and medical appearance of medical products also attracts unwanted attention, making patients feel self-conscious and reducing their sense of normalcy. These factors negatively affect mental health and overall quality of life.

The present disclosure aims to mitigate one or more of the above challenges by providing a carrycase comprising a carrycase body for housing the medical product. The carrycase body comprises a carrycase opening, which may be closable by means of a closing mechanism. The closure mechanisms may for example be a magnetic closure, snap fastening, hook-and-loop fastener, or other suitable mechanisms for securing the opening.

The shoulder strap may be configured to carry the carrycase like a handbag, preferably as a cross-body bag, offering a casual and aesthetically integrated appearance. The presence of a shoulder strap furthermore provides ergonomic comfort, stability and easy access to the medical product. When the carrycase is configured as a cross-body bag, the shoulder strap enhances security by reducing the likelihood of accidentally slipping and improves mobility, making it easier to walk, travel or engage in daily activities. The shoulder strap may be in the form of a closed loop or constitute of two strap members that can be connected to form a closed loop, such as by tying a knot or in that one or both strap members is/are provided with a fastener, such as a buckle, Velcro fastening, snap fastener or the like. A shoulder strap constituting of two strap members may provide a multi-purpose strap, such that the strap members may alternatively be tied around the waist and the carrycase be carried as a waist bag.

The elongated tube-shaped portion having a central through opening extending from the carrycase body provides a separate casing for a tubing connected to the medical product, thereby preventing the tubing from folding and ensuring improved functionality. The elongated tube-shaped portion, open in both ends, facilitates connection of the tubing, such as to a wound site for a negative pressure wound therapy device.

In exemplary embodiments, the carrycase case is made of an elastic material that ensures a snug, non-bulky fit for the carrycase body, while maintaining comfort and easy access to the medical product. The fact the carrycase is made of an elastic material provides both functionality and aesthetics, allowing the tube-shaped portion to integrate seamlessly with the clothing of the patient. The fact that the carrycase is made of an elastic material furthermore provides for an easy to manufacture and cost-efficient portable solution.

By "elastic material" herein is intended a material that can deform, at least in the transverse direction, under an applied force, such as stretching, and essentially returns to its original shape and size when the force is removed. Examples of suitable elastic materials for the carrycase are knitted material, elastic nonwoven materials, elastic woven material and spacer fabrics.

The elastic material may, for examples, be made of fibers, such as polyester fibers, nylon fibers, polyurethane fibers, natural fibers and manmade cellulosic fibers.

In exemplary embodiments, the carrycase is made of a knitted material, optionally a continuous knitted material. Optionally, the elongated tube-shaped portion is made of the knitted material, such as the continuous knitted material. Optionally, the carrycase body and the elongated tube-shaped portion are made of the knitted, such as the continuous knitted material. In one embodiment, the carrycase body, the shoulder strap and the elongated tube-shaped portion are all made of the knitted material.

In exemplary embodiments, the carrycase body, the shoulder strap and the elongated tube-shaped portion are made of the continuous knitted material such that the carrycase body, the shoulder strap and the elongated tube-shaped portion constitutes one continuously knitted unit. This provides for an aesthetically pleasing design which is also cost-efficient and simple to manufacture since the carrycase may be manufacture in a single knitting procedure.

By "continuous knitting" refers to a knitting which results in a continuous unbroken structure without the need for seams or joins. This includes techniques where yarns are looped in a circular or three-dimensional configuration, such as on circular knitting machines for tubular fabrics or advanced knitting machines for the fully-shaped carrycase. In this context, the carrycase may thus be knitted as a tubular unit, with varying diameter. The section forming the carrycase body may transition into two narrower tube-shaped sections corresponding to the shoulder strap, which may merge during the knitting process or by incorporating a seam forming one closed loop.

In exemplary embodiments, the carrycase, including the carrycase body, the shoulder strap and the elongated tube-shaped portion, is made of a tubular knit fabric.

In exemplary embodiments, the elastic material is a spacer fabric. The use of a spacer fabric for the present carrycase may offer several advantages. A carrycase made by a spacer fabric may provide improved protection of the medical product, reduced visibility through the fabric and provide for a sound-dampening effect, all while maintaining a flexible structure. A sound-dampening effect may be particularly beneficial for specific medical products, such as for example a portable NPWT devices. Additionally, a spacer fabric may reduce the risk of accidentally pressing buttons or otherwise manipulating the device, ensuring safer handling and operation.

A spacer fabric is a three-dimensional textile structure composed of two outer fabric layers connected by a network of spacer yarns or filaments. These connecting filaments create a space or gap between the layers, providing the fabric with thickness, cushioning and durability.

In exemplary embodiments, the elastic material has a no-load thickness within the range of from 1 mm to 5 mm, as measured according to ASTM D177-96(2019).

In exemplary embodiments, the elastic material has a no-load thickness within the range of from 1 mm to 5 mm in the section forming the carrycase body, as measured according to ASTM D177-96(2019).

The use of an elastic material having a no-load thickness within the range of from 1 mm to 5 mm provides for a cushioning effect offering enhanced protection for the medical product against impacts. Additionally, the thickness mentioned herein also reduces the visibility and contributes to a more discreet and aesthetically pleasing appearance. The thickness mentioned herein also improves the overall durability and structural integrity of the carrycase.

In exemplary embodiments, the elastic material of the carrycase body has a greater no-load thickness than the elastic material of the elongated tube-shaped portion, as measured according to ASTM D177-96(2019), optionally at least 20% greater.

In exemplary embodiments, the elastic material has a no-load thickness within the range of from 1 mm to 3 mm in the section forming the elongated tube-shaped portion, as measured according to ASTM D177-96(2019).

The fact that the no-load thickness of the elastic material is greater for the carrycase body than for the elongated tube-shaped portion provides a cushioning effect offering enhanced protection for the medical product against impacts, while maintaining flexibility for the elongated tube-shaped portion.

The no-load thickness of the elastic material may vary between the carrycase body, the shoulder strap and the elongated tube-shaped body, even though the material is knitted as a single continuous unit.

In exemplary embodiments, the shoulder strap has a total length of at least 800 mm, optionally within the range of from 900 mm to 2000 mm, as measured in the longitudinal direction of the carrycase and from a respective end point of the shoulder strap. The carrycase may be a cross-body bag, being defined by a cross-body shoulder strap being longer than a conventional shoulder strap.

In alternative embodiments, the shoulder strap may be adapted to be worn around the waist of a user, such that the bag additionally may be used as a waist bag.

In exemplary embodiments, the shoulder strap has a width within the range of from 10 mm to 60 mm, as measured in the transverse direction of the carrycase and in a non-stretched state.

In exemplary embodiments, the carrycase body has a length within the range of from 140 mm to 400 mm, optionally within the range of from 180 to 350 mm, as measured in the longitudinal direction of the carrycase and in an unstretched state of the carrycase.

In exemplary embodiments, the carrycase body has a width within the range of from 80 mm to 200 mm, as measured in the transverse direction of the carrycase and in an unstretched state of the carrycase body.

In exemplary embodiments, the elongated tube-shaped portion has a length within the range of from 250 mm to 2000 mm, optionally within the range of from 500 mm to 1500 mm, as measured in the longitudinal direction of the carrycase,

In exemplary embodiments, the elongated tube-shaped portion has a width is within the range of from 5 mm to 50 mm, as measured in the transverse direction of the carrycase and in an unstretched state of the elongated tube-shaped portion.

In exemplary embodiments, the shoulder strap extends from an upper portion of the carrycase body and the elongated tube-shaped portion extends from a lower portion of the carrycase body, the upper and lower portions being opposing portions of the carrycase body as seen in the longitudinal direction.

The configuration when the elongated tube-shaped portion extends from the lower portion of the carrycase body is particularly advantageous for a carrycase intended for a portable NPWT device comprising a negative pump and a tubing. This allows for the tubing to extend downwards from the negative pressure pump, reducing the risk of wound exudate pooling. Additionally, it minimizes the risk of fluid backflow into the wound or the pump, especially if the pump experiences interruptions.

In exemplary embodiments, the elastic material has a transverse percentage elongation in the transverse direction of the carrycase, and a longitudinal percentage elongation in the longitudinal direction Y of the carrycase, and wherein the transverse percentage elongation is greater than the longitudinal percentage elongation.

The term "percentage elongation" refers to a percentage of an increase in length/width from a non-stretched state to a fully stretched state. Percent elongation is calculated by dividing the change in length/width by the length/width in the non-stretched state and multiplying by 100.

A greater transverse percentage elongation compared to the longitudinal percentage elongation enhances the flexibility and adaptability of the carrycase body and the elongated tube-shaped portion, allowing the carrycase to achieve a less bulky appearance while conforming better to irregularly shaped medical products. Additionally, it maintains structural stability by preventing the carrycase from stretching or sagging excessively under the weight of the medical product. This ensures the product remains securely positioned within the carrycase, contributing to both an ergonomic and comfortable fit for the user.

In exemplary embodiments, the transverse percentage elongation of the elastic material is at least 20% greater than the longitudinal percentage elongation of the elastic material.

In exemplary embodiments, the transverse percentage elongation of the elastic material is within the range of from 25% to 400%, preferably within the range from 40% to 350%, more preferably within the range of from 40% to 300%.

In exemplary embodiments, the longitudinal percentage elongation of the elastic material is within the range of from 0% to 150%, preferably within the range of from 0% to 100%, more preferably within the range of from 0% to 75%, or within the range of from 0% to 50%, or within the range of from 0% to 25%.

In exemplary embodiments, the elastic material has a first transverse percentage elongation in the section forming the shoulder strap, the first transverse percentage elongation being within the range of from 50% to 300%, preferably within the range of from 75% to 250%, more preferably within the range of from 100% to 200%.

In exemplary embodiments, the elastic material has a second transverse percentage elongation in the section forming the carrycase body, the second transverse percentage elongation being within the range of from 25% to 100%, preferably within the range of from 30% to 75%.

In exemplary embodiments, the elastic material has a third transverse percentage elongation, in the transverse direction of the carrycase, in the section forming the elongated tube-shaped portion, the third transverse percentage elongation being within the range of from 100% to 400%, preferably within the range of from 100% to 250%.

The elasticity of the elastic material may vary between the carrycase body, the shoulder strap and the elongated tube-shaped body portion, even though the material is knitted as a single continuous unit.

According to a second aspect a kit is provided, wherein the kit comprises a carrycase according to a first aspect and a medical product.

In exemplary embodiments, the medical product comprises a pump and a tubing connected to the pump.

In exemplary embodiments the medical product is a portable NPWT device comprising a negative pressure pump and a tubing connecting to the negative pressure pump.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated.

### BRIEF DESCRIPTION OF THE DRAWINGS

Aspects and embodiments are now described, by way of example, with reference to the accompanying drawings, in which:
Fig 1 shows a user carrying the carrycase as disclosed herein.
Fig 2 is a plan view of the carrycase.
Fig 3 illustrates a portable medical product suitably carried by the carrycase.
Fig 4 is cross-sectional view of a carrycase according to the present disclosures.

### DETAILED DESCRIPTION

The aspects of the present disclosure will now be described more fully hereinafter with reference to the accompanying drawings, in which certain embodiments of the invention are shown.

These aspects may, however, be embodied in many different forms and should not be construed as limiting; rather, these embodiments are provided by way of example so that this disclosure will be thorough and complete, and to fully convey the scope of all aspects of invention to those skilled in the art. Like numbers refer to like elements throughout the description.

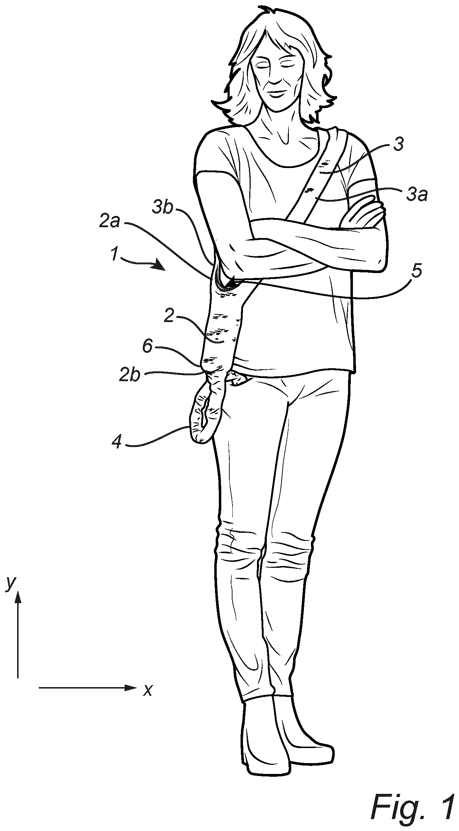

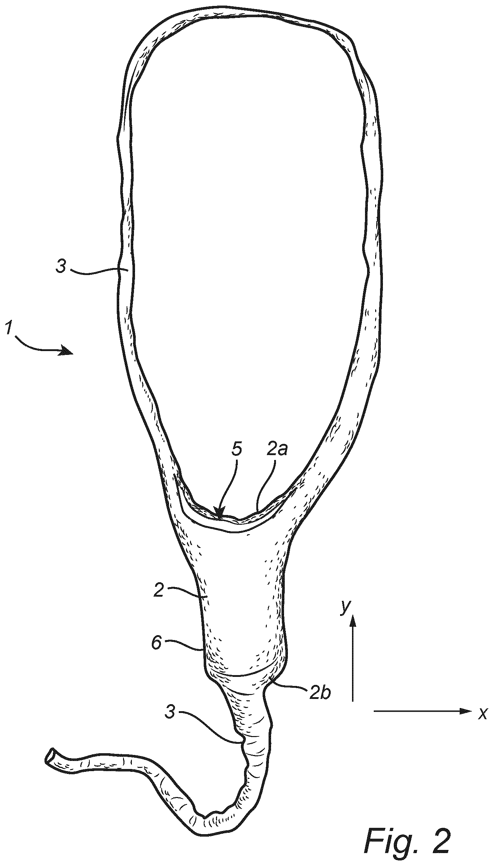

Figure 1 illustrates a user carrying the carrycase 1 as disclosed herein. Figure 2 is a plan view of the carrycase 1 shown in Figure 1.

The carrycase 1 comprises a carrycase body 2, a shoulder strap 3 for carrying the carrycase 1 and an elongated tube-shaped portion 4 extending from the carrycase body 2. The carrycase 1 extends in a longitudinal direction Y and a transverse direction X.

The carrycase 1 may, as illustrated in Figure 1, be configured to be worn as a cross-body bag. A cross-body bag is defined by a shoulder strap that is either longer than a standard shoulder strap or adjustable in length.

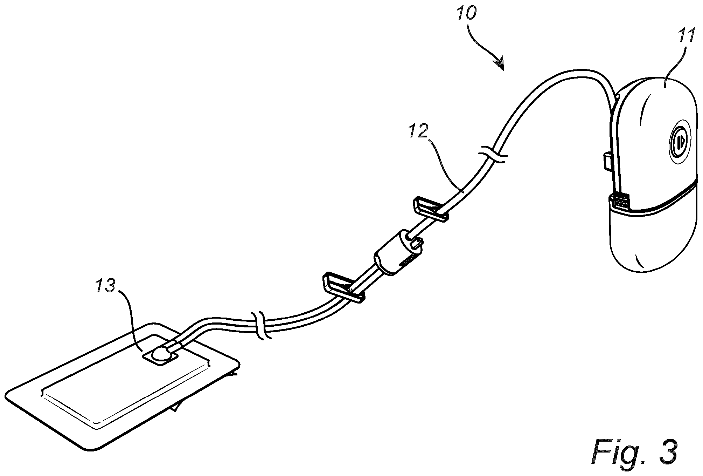

The shoulder strap extends from an upper portion 2a of the carrycase body 2 and the elongated tube-shaped portion 4 extends from a lower portion 2b of the carrycase body 2. As shown in Figure 1, the upper and lower portions are opposing portions of the carrycase body 2 as seen in the longitudinal direction Y. The carrycase body 2 comprises a carrycase opening 5 at the upper portion 2a, to insert or remove a medical product 10, shown in Figure 3, from the carrycase 1.

The configuration when the elongated tube-shaped portion 3 extends from the lower portion 2b of the carrycase body 2 is particularly advantageous for a carrycase 1 when the medical product 10 is a portable NPWT device comprising a negative pump 11 and a tubing 12, shown in Figure 3. This allows the tubing 12 to extend downwards from the negative pressure pump 11, reducing the risk of wound exudate pooling. Additionally, it minimizes the risk of fluid backflow into the wound 13 or the pump, especially if the pump experiences interruptions.

The carrycase 1 is made of an elastic material 6. In these figures, the elastic material is a continuous knitted material, such as a circular knitted material. In the carrycase 1 illustrated, the carrycase body 2, the shoulder strap 3 and the elongated tube-shaped portion 4 are continuously knitted as a tube in one unit with varying diameter and with the section forming the carrycase body 2 transitioning into two narrower tube sections 3a, 3b corresponding to the shoulder strap 3, which merge during the knitting process, i.e., without a seam. Alternatively, the narrow tube sections 3a,3b are joined by a seam to form a closed loop.

In Figure 3, the medical product 10 is exemplified as a negative pressure wound therapy (NPWT) device. The NPWT device 10 comprises a negative pressure pump 11 and a tubing 12 connected to the negative pressure pump 11 in one end and to a medical dressing 13, arranged to cover a wound site in an opposing end.

When the portable medical product 10 is a portable NPWT device 10, the negative pressure pump 11 is suitably arranged in the carrycase body 2 and the tubing 12 in the elongated tube-shaped portion 4. The tube-shaped portion 4 is designed and dimensioned such that air-inlet devices and flow-control devices on the tubing 12 may be fitted inside the tube-shaped portion 4. The tube-shaped portion 4 may be made of a material/fabric which allows air to enter air inlets along the tubing.

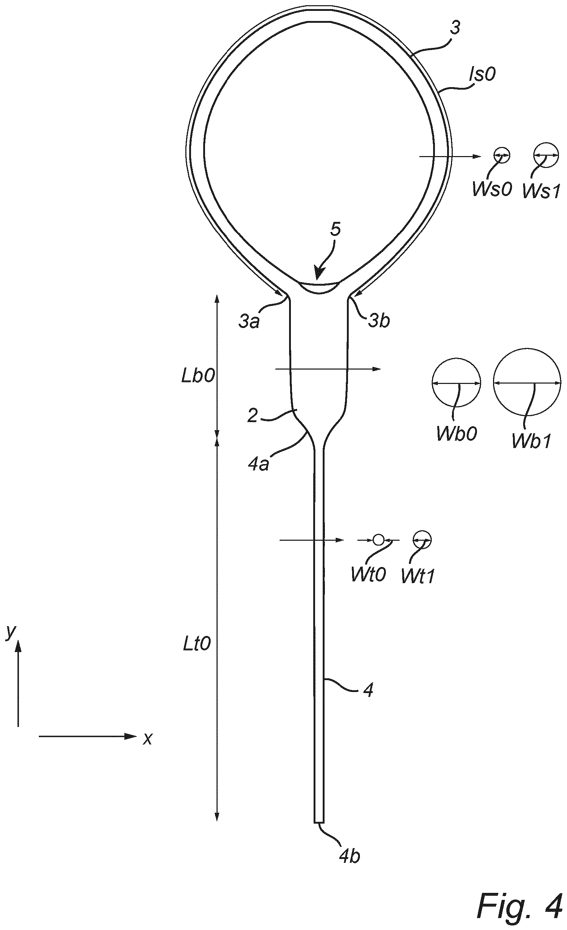

Figure 4 illustrates a cross-sectional view of the carrycase 1.

A length Lso of the shoulder strap 3 of the carrycase 1 is at least 800 mm, optionally within the range of from 900 mm to 2000 mm, and is measured between a respective end point 3a,3b of the shoulder strap 3, or a respective transition point with the carrycase body 2, and at a non-stretched state.

The shoulder strap 3 in Figure 4 has an initial, non-stretched, width Wso within the range of from 10 mm to 60 mm, as measured in the transverse direction X of the carrycase 1.

The shoulder strap 3 may have a fully stretched width Wₛ1 within the range of from 15 mm to 240 mm, as measured in the transverse direction X of the carrycase 1.

The elastic material in the shoulder strap 3 may have a first transverse percentage elongation of from 50% to 300%, preferably within the range of from 75% to 250%. The first transverse percentage elongation is calculated by dividing the change in width, i.e. from the initial width Wso to the fully stretched width Ws1, with the unstretched width Wso and multiplying by 100.

The carrycase body 2 illustrated in Figure 4 has a length Lbo within the range of from 140 mm to 400 mm, optionally within the range of from 180 to 350 mm, as measured in the longitudinal direction Y of the carrycase and in an unstretched state.

The carrycase body 2 may have an initial width Wbo within the range of from 80 mm to 200 mm, as measured in the transverse direction of the carrycase 1 and in a non-stretched state.

The width wb1 of the carrycase body 2 in a fully stretched state may be within the range of from 100 mm to 400 mm, as measured in the transverse direction of the carrycase 1.

The elastic material in the carrycase body 2 may have a second transverse percentage elongation of from 25% to 100%, preferably within the range of from 30% to 75%. The second transverse percentage elongation is calculated by dividing the change in width, i.e. from the initial width Wb0 to the fully stretched width Wb1, with the unstretched width Wb0 and multiplying by 100.

In exemplary embodiments, the length Lto of the elongated tube-shaped portion 4, as measured between a first end 4a and a second end 4b, is within the range of from 250 mm to 2000 mm, optionally within the range of from 500 mm to 1500 mm, as measured in the longitudinal direction Y of the carrycase 1. The first end of the elongated tube-shaped portion 4 corresponding to the opening into the carrycase body 2 and the second end 4b, being an open end 4b of the elongated tube-shaped portion 4.

An initial width Wₜ0 of the elongated tube-shaped portion 4 may be within the range of from 5 mm to 50 mm, as measured in the transverse direction X of the carrycase 1. A width Wt1 in the fully stretched state may be within the range of from 10 mm to 200 mm.

The elastic material in the elongated tube-shaped portion 4 may have a third transverse percentage elongation of from 100% to 400%, preferably within the range of from 100% to 250%. The third transverse percentage elongation is calculated by dividing the change in width, i.e. from the initial width Wt0 to the fully stretched width Wt1, with the unstretched width Wt1 and multiplying by 100.

The elasticity of the elastic material may thus vary between the carrycase body 2, the shoulder strap 3and the elongated tube-shaped body 4, even though the material is knitted as a single continuous unit.

## Claims

1. A carrycase (1) for a portable medical product (10), the carrycase extends in a longitudinal direction (Y) and in a transverse direction (X), the carrycase comprises a carrycase body (2), a shoulder strap (3) for carrying the carrycase and an elongated tube-shaped portion (4) extending from the carrycase body, wherein the carrycase is made of an elastic material (6).

2. The carrycase according to claim 1, wherein the carrycase is made of a knitted material, optionally a continuous knitted material.

3. The carrycase according to claim 2, wherein the carrycase body, the shoulder strap and the elongated tube-shaped portion are made of the continuous tubular knitted material.

4. The carrycase according to any one of the preceding claims, wherein the elastic material is a spacer fabric.

5. The carrycase according to any one of the preceding claims, wherein the elastic material has a no-load thickness within the range of from 1 mm to 5 mm, as measured according to ASTM D177-96(2019).

6. The carrycase according to any one of the preceding claims, wherein the shoulder strap has a total length (Lso) of at least 800 mm, optionally within the range of from 900 mm to 2000 mm, as measured in the longitudinal direction (Y) of the carrycase, and/or a width (wso) within the range of from 10 mm to 60 mm, as measured in the transverse direction (X) of the carrycase.

7. The carrycase according to any one of the preceding claims, wherein the carrycase body has a length (Lbo) within the range of from 140 mm to 400 mm, as measured in the longitudinal direction (Y) of the carrycase, and/or a width (Wbo) within the range of from 80 mm to 200 mm, as measured in the transverse direction (X) of the carrycase.

8. The carrycase according to any one of the preceding claims, wherein the length (Lt0) of the elongated tube-shaped portion is within the range of from 250 mm to 2000 mm, as measured in the longitudinal direction (Y) of the carrycase, and/or a width (Wto) within the range of from 5 mm to 50 mm, as measured in the transverse direction (X) of the carrycase.

9. The carrycase according to any one of the preceding claims, wherein the shoulder strap extends from an upper portion (2a) of the carrycase body and the elongated tube-shaped portion extends from a lower portion (2b) of the carrycase body, the upper and lower portions being opposing portions of the carrycase body as seen in the longitudinal direction (Y).

10. The carrycase according to any one of the preceding claims, wherein the elastic material has a transverse percentage elongation in the transverse direction (X) of the carrycase, and a longitudinal percentage elongation in the longitudinal direction (Y) of the carrycase, and wherein the transverse percentage elongation is greater than the longitudinal percentage elongation.

11. The carrycase according to claim 10, wherein the transverse percentage elongation is within the range of from 25% to 400%.

12. The carrycase according to claims 10 or 11, wherein the longitudinal percentage elongation is within the range of from 0% to 150%.

13. The carrycase according to any one of the preceding claims, wherein the elastic material has a first transverse percentage elongation in the section forming the shoulder strap, the first transverse percentage elongation being within the range of from 50% to 300%.

14. The carrycase according to any one of the preceding claims, wherein the stretchable or elastic material has a second transverse percentage elongation in the section forming the carrycase body, the second transverse percentage elongation being within the range of from 25% to 100%.

15. The carrycase according to any one of the preceding claims, wherein the elastic material has a third transverse percentage elongation, in the transverse direction (X) of the carrycase, in the section forming the elongated tube-shaped portion, the third transverse percentage elongation being within the range of from 100% to 400%.
